# EUROPÄISCHE PATENTSCHRIFT

(11) **EP 2 096 101 B1**
(45) Veröffentlichungstag und Bekanntmachung des Hinweises auf die Patenterteilung: **18.04.2012**
(21) Anmeldenummer: 09153525.2
(22) Anmeldetag: 24.02.2009
(51) Int. Cl.: C07C 253/30, C07C 221/00, C07C 45/68, C07C 255/22, C07C 255/56, C07D 213/50, C07D 307/46, C07D 333/22

(54) **Verfahren zur Herstellung von Ketonen aus alpha-Oxocarboxylaten und Arylbromiden**
Method for producing ketones from alpha-oxocarboxylates and aryl bromides
Procédé de fabrication de kétones à partir d'alpha-oxocarboxylates et d'arylbromides

(30) Priorität: 28.02.2008 DE 102008011685
(43) Veröffentlichungstag der Anmeldung: 02.09.2009
(73) Patentinhaber: Saltigo GmbH, 40764 Langenfeld (DE)
(72) Erfinder: Cotté, Alain, 51377, Leverkusen (DE); Gotta, Matthias, 51061, Köln (DE); Goossen, Lukas, 67663, Kaiserslautern (DE); Rudolphi, Felix, 67663, Kaiserslautern (DE); Oppel, Christoph, 67661, Kaiserslautern (DE); Garrido, Nuria Rodriguez, 67663, Kaiserslautern (DE)
(74) Vertreter: Pettrich, Klaus-Günter

(56) Entgegenhaltungen:
- WO-A-2006/136135
- GOOSSEN, L.J. ET AL: "Pd-Catalyzed Syntheis of Functionalized Arylketones from Boronic Acids and Carboxylic Acids ACtivated in situ with Dimethyl Dicarbonate" SYNLETT, Nr. 8, 2002, Seiten 1237-1240, XP002527729 Stuttgart
- NILSSON M: "A NEW BIARYL SYNTHESIS ILLUSTRATING A CONNECTION BETWEEN THE ULLMANN BIARYL SYNTHESIS AND COPPER-CATALYSED DECARBOXYLATION" ACTA CHEMICA SCANDINAVICA, MUNKSGAARD, COPENHAGEN, DK, Bd. 20, Nr. 2, 1. Januar 1966 (1966-01-01), Seiten 423-426, XP009075993 ISSN: 0904-213X
- TAKEMIYA, A.; HARTWIG, J.F.: "Palladium-Catalyzed Synthesis of Aryl Ketones by Coupling of Aryl Bromides with an Acyl Anion Equivalent" JOURNAL OF THE AMERICAN CHEMICAL SOCIETY, Bd. 128, Nr. 46, 2006, Seiten 14800-14801, XP002527730 Washington, USA

## Beschreibung

Die vorliegende Erfindung betrifft ein neues Verfahren zur Herstellung von Ketonen aus alpha-Oxocarboxylaten und Arylbromiden durch Cu/Pd-katalysierte decarboxylierende Kreuzkupplung.

Arylketone sind wichtige Strukturelemente in Wirkstoffen und funktionellen Materialien (P. J. Masson, D. Coup, J. Millet, N. L. Brown, J. Biol. Chem. 1994, 270, 2662-2668). Neben der klassische Friedel-Crafts-Acylierungen die im großtechnischen Maßstab durchgeführt werden, aber meist nachteilig zu Isomerengemische führen, werden zu ihrer Darstellung ebenfalls Umsetzungen aktivierter Carbonsäurederivate mit metallorganischen Reagenzien genutzt wie z.B. Weinreb-Amide mit Grignard-Verbindungen (S. Nahm, S. M. Weinreb, Tetrahedron Lett. 1981, 22, 3815-3818). Durch die Verwendung von Übergangsmetallkatalysatoren lässt sich die Effizienz solcher Kreuzkupplungen weiter erhöhen, sodass selbst wenig reaktive Kohlenstoffnucleophile umgesetzt werden, beispielsweise Organozinkverbindungen oder Boronsäuren (E. Negishi, V. Bagheri, S. Chatterjee, F. T. Luo, J. A. Miller, A. T. Stoll, Tetrahedron Lett. 1983, 24, 5181-5184 ;P. Knochel, M. C. P. Yeh, S. C. Berk, J. Talbert, J. Org. Chem. 1988, 53, 2392-2394; M. Haddach, J. R. McCarthy, Tetrahedron Lett. 1999, 40, 3109-3112).

Bequemer sind Reaktionsvarianten, bei denen Carbonsäuren *in situ* aktiviert werden, wie z.B. bei palladiumkatalysierten Direktsynthesen von Arylketonen aus Arylboronsäuren und Carbonsäuren in Gegenwart von Anhydriden (L. J. Gooßen, L. Winkel, A. Döhring, K. Ghosh, J. Paetzold, Synlett 2002, 8, 1237-1240) oder Kupplungsreagenzien (L. J. Gooßen, K. Ghosh, Chem. Comm. 2001, 2084-2085). Durch diese Methoden wird die Toleranz funktioneller Gruppen zwar deutlich verbessert jedoch hat diese Vorgehensweise aufgrund der schwierigen Zugänglichkeit von Boronsäuren (Metallierung von Arylhalogeniden und nachfolgende Umsetzung mit Trialkylboraten) oder wegen der schlechte Handhabung von Organo-Zink-Verbindungen gravierende Nachteile.

Eine umgekehrte Vorgehensweise, bei der Acylanionen-Äquivalente mit Kohlenstoffelektrophilen gekuppelt werden, wird vorwiegend bei der Synthese von Alkylketonen eingesetzt (E. J. Corey, D. Seebach, Angew. Chem. 1965, 77, 1134-1135; Angew. Chem. Int. Ed. 1965, 4, 1075-1077). Für die katalytische Arylierung von Acylanionen-Äquivalenten gibt es nur wenige Beispiele, darunter die von Hartwig et al. beschriebene Kupplung von Arylbromiden mit *N-tert*-Butylhydrazonen (A. Takemiya, J. F. Hartwig, J. Am. Chem. Soc. 2006, 128, 14800-14801). Nachteilig bei allen Reaktionen dieses Typs sind die zusätzlichen Derivatisierungs- und Verseifungsschritte, sowie die Verwendung starker Basen. Arylierungen von Aldehyden unter C-H-Aktivierung bieten eine atomökonomische Alternative, sind aber bisher nur mit einem eingeschränkten Spektrum an teuren Aryliodiden möglich (Y. C. Huang, K. K. Majumdar, C. H. Cheng, J. Org. Chem. 2002, 67, 1682-1684; S. Ko, B. Kang, S. Chang, Angew. Chem. 2005, 117, 459-461; Angew. Chem. Int. Ed. 2005, 44, 455-457).

Bei der Biarylsynthese aus Benzoesäuresalzen und Arylhalogeniden gelang bereits der Nachweis, dass decarboxylierende Kreuzkupplungen wertvolle Alternativen zu entsprechenden Umsetzungen von Organometall-Verbindungen darstellen können (WO 2006/136135).

Aufgabe der vorliegenden Erfindung war es, ein Verfahren zur Herstellung von Ketonen, insbesondere Arylketonen, aufzufinden, bei dem ausgehend von gut zugänglichen Verbindungen gut handhabbare und preiswerte Katalysatoren zum Einsatz kommen.

Wird die Decarboxylierung von alpha-Oxocarbonsäuren der Formel (I) in Anwesenheit eines Cu-Katalysators durchgeführt und mit einer Pd-vermittelten Kreuzkupplung mit Arylbromiden der Formel (II) kombiniert, gelangt man zu den Ketonen der Formel (III):

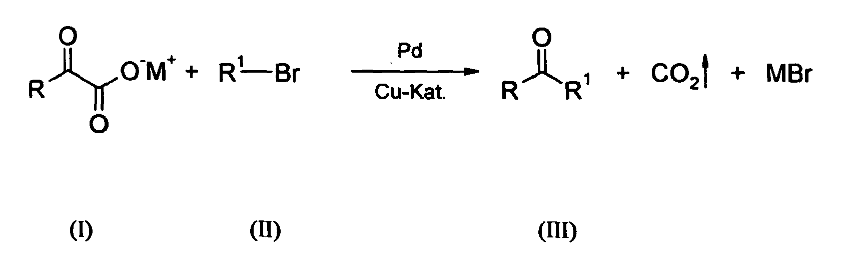

Vorteil dieser decarboxylierenden Kreuzkupplung zur Herstellung von Ketonen ist, dass im Gegensatz zu traditionellen Kreuzkupplung-Reaktionen keine metallorganischen Reagenzien erforderlich sind. Stattdessen werden einfach handhabbare, gut zugängliche stabile Salze von alpha-Oxocarbonsäuren, die zum Teil als Zwischenprodukte bei der Herstellung von Aminosäuren großtechnisch verfügbar sind, als Quelle von Acylnucleophilen genutzt.

Gegenstand der Erfindung ist daher ein Verfahren zur Herstellung von Ketonen der allgemeinen Formel (III) 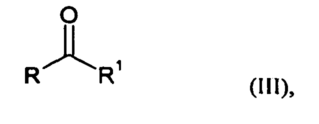 wobei
- R: ein gegebenenfalls substituierter carbocyclischer aromatischer Rest mit 6 bis 24 Kohlenstoffatomen oder ein gegebenenfalls substituierter Alkylrest oder ein gegebenenfalls substituierter heteroaromatischer Rest mit 5 bis 24 Kohlenstoffatomen ist und
- R¹: ein gegebenenfalls substituierter carbocyclischer aromatischer Rest mit 6 bis 24 Kohlenstoffatomen oder ein gegebenenfalls substituierter heteroaromatischer Rest mit 5 bis 24 Kohlenstoffatomen ist,
durch Umsetzung von alpha-Oxocarboxylaten der allgemeinen Formel (I) 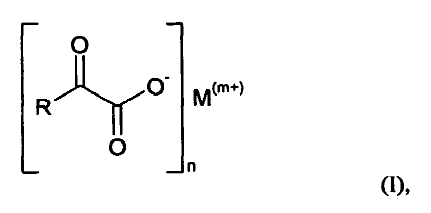 wobei
n und m eine Zahl im Bereich von 1 bis 6 ist,
- M^{(m+)}: ein Kation ist und
- R: die für Formel (III) angegebene Bedeutung hat,
mit Arylbromiden der allgemeinen Formel (II)

R¹-Br (II)

wobei
- R¹: die für Formel (III) angegebene Bedeutung hat,
wobei als Katalysator eine Kombination aus Kupfer(I)bromid mit 1,10-Phenanthrolin als Ligand und Bis(1,1,1,5,5,5-hexafluoroacetylacetonato)palladium mit Tris(o-tolyl)phoshin als Ligand eingesetzt wird.

Durch Zusatz des Phosphinliganden wird die Umsetzung vorteilhaft beeinflusst. Es wird Tris(o-tolyl)phosphin ((*o*-Tol)₃P) eingesetzt.

Für das erfindungsgemäße Verfahren wird als Katalysatorsystem eine Kombination aus Kupfer(I)bromid mit 1,10-Phenanthrolin als Ligand und Pd(F₆acac)₂ mit Tris(o-tolyl)phosphin als Ligand eingesetzt.

Gemäß dem erfindungsgemäßen Verfahren werden beide Katalysatoren unabhängig voneinander in Mengen von 0,001 mol% bis 100 mol% bezogen auf das Kohlenstoffelektrophil (R¹-Br) eingesetzt, bevorzugt werden Mengen von 0,01 mol% bis 15 mol% eingesetzt.

Das erfindungsgemäße Verfahren wird bei Temperaturen von 20 °C bis 220 °C, vorzugsweise bei 80 °C bis 200 °C und besonders bevorzugt bei 120 °C bis 180 °C durchgeführt.

Das erfindungsgemäße Verfahren wird üblicherweise in Gegenwart eines Lösungsmittels durchgeführt. Beispielsweise können als Lösungsmittel einer oder Gemische der Einsatzstoffe, aromatische Kohlenwasserstoffe (beispielsweise Benzol, Toluol, Xylole, Ethylbenzol, Mesitylen), Ether (beispielsweise 1,4-Dioxan, Tetrahydrofuran, Methyltetrahydrofuran, Dibutylether, Methyl-t-butylether, Diisopropylether, Diethylenglycol-dimethylether), Amide (beispielsweise Dimethylformamid, Diethylformamid, N-Methylpyrrolidon, Dimethylacetamid), aromatische Amine (Chinolin, Pyridin), Dimethylsulfoxid, Sulfolan, Acetonitril, Isobutyronitril, Propionitril, Propylencarbonat und chlorierte aliphatische und aromatische Kohlenwasserstoffe eingesetzt werden. Bevorzugt werden Amide (beispielswiese Dimethylformamid, N-Methylpyrrolidon) und aromatische Amine (beispielsweise Chinolin, Pyridin) oder Gemische Amide/Amine eingesetzt.

Das erfindungsgemäße Verfahren kann vorzugsweise so durchgeführt werden, dass während der Reaktion Spuren von Wasser durch kontinuierlich azeotrope Destillation entfernt werden können. Zur Erreichung der erforderlichen Reaktionstemperatur kann auch unter Druck gearbeitet werden.

R und R¹ stehen im Rahmen der Erfindung bevorzugt für carbocyclische aromatische Reste mit 6 bis 24 Gerüstkohlenstoffatomen oder heteroaromatische Reste mit 5 bis 24 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können. Weiterhin können die carbocyclischen aromatischen Reste oder heteroaromatische Reste mit bis zu fünf gleichen oder verschiedenen Substituenten pro Cyclus substituiert sein, ausgewählt aus der Gruppe Hydroxy, Halogen, Nitro, Amino, Mercapto, Cyano, freies oder geschütztes Formyl, C₁-C₁₂-Alkyl, C₁-C₁₂-Halogenalkyl, C₁-C₁₂-Alkylthio; C₅-C₁₄-Aryl, C₆-C₁₅-Arylalkyl, C₁-C₁₂-Alkoxy und C₁-C₁₂-Alkoxycarbonyl. Beispielsweise steht R¹ für Phenyl, Tolyl, Thienyl oder Naphthyl, das gegebenenfalls einfach, zweifach oder dreifach durch Reste substituiert ist, die jeweils unabhängig voneinander ausgewählt sind aus der Gruppe C₁-C₆-Alkyl, C₁-C₆-Halogenalkyl, C₁-C₆-Alkylthio, C₅-C₁₄-Aryl, C₁-C₆-Alkoxy, C₁-C₆-Alkoxycarbonyl, Halogen, Hydroxy, Nitro, Amino, Mercapto und Cyano. Bevorzugt ist R¹ ein Tolyl-, Cyanophenyl- oder Methoxyphenylrest.

Zusätzlich steht R im Rahmen der Erfindung bevorzugt für Alkyl jeweils unabhängig für einen gegebenenfalls substituierten geradkettigen, cyclischen, verzweigten oder unverzweigten Alkyl-Rest. Als Substituenten für den Alkyl-Rest kommen beispielsweise C₁-C₆-Alkyl, C₁-C₆-Halogenalkyl, C₅-C₁₄-Aryl, C₆-C₁₅-Arylalkyl, C₁-C₆-Alkoxy, C₁-C₆-Aryloxy, C₁-C₆-Alkoxycarbonyl, C₁-C₆-Acyloxy oder Halogen in Frage. Beispielsweise steht Alkyl besonders bevorzugt für n-Propyl, Isopropyl, n-Butyl, tert.-Butyl, iso-Butyl, n-Pentyl, oder Cyclohexyl. Bevorzugt ist R ein Phenyl-, Cyanophenyl- oder tert.-Butylrest.

Das Gegenion M^{(m+)} aus der Formel (I) ist dabei ein Metallkation, das zum Ladungsausgleich führt, bevorzugt aus der Reihe der Metalle Li, Na, K, Mg, Ca, B, Al, Ag, Cu, Mn, Fe, Co, Ni, Mo, Ru, besonders bevorzugt aus der Reihe Natrium oder Kalium (n = 1).

Die alpha-Oxocarbonsäuresalze der Formel (I) werden wahlweise in präformierter Form zugesetzt oder in situ aus den alpha-Oxocarbonsäuren und geeigneten Basen erzeugt.

### Beispiele

### Beispiel 1: Herstellung von tert-Butyl-(4-tolyl)-keton.

In einem Reaktionsgefäß wurden Kalium-3,3,3-trimethylpyruvat (5.05 g, 30.0 mmol), Bis(1,1,1,5,5,5-hexafluoracetylacetonato)palladium (104.1 mg, 0.20 mmol) und Kupfer(I)bromid (430.4 mg, 3.00 mmol) unter Stickstoff vorgelegt und mit einer Lösung von 4-Bromtoluol (3.42 g, 2.46 mL, 20 mmol), Tris(o-tolyl)phosphin (182.6 mg, 0.6 mmol) und 1,10-Phenanthrolin (541 mg, 3.0 mmol) in 36 mL NMP und 8.5 mL Pyridin versetzt. Die Reaktionsmischung wurde für 36 Stunden unter Rückfluss erhitzt (170 °C), nach dem Abkühlen durch Celite filtriert und der Filterkuchen mit Diethylether nachgewaschen. Die erhaltene Lösung wurde dreimal mit 1 M Salzsäure gewaschen und die wässrigen Phasen zweimal mit Diethylether extrahiert. Die vereinigten organischen Phasen wurden mit 100 mL gesättigter Natriumchloridlösung gewaschen, über Magnesiumsulfat getrocknet und filtriert. Nach Entfernen des Lösungsmittels wurde das Produkt mittels Kugelrohrdestillation gereinigt (105 °C / 4 x 10⁻³ mbar) und als gelbes Öl erhalten (3.17 g, 90% Ausb.). Die spektroskopischen Daten entsprechen denen von *tert*-Butyl-(4-tolyl)keton.

### Beispiel 2: Herstellung von 4-Methylbenzophenon.

In einem Reaktionsgefäß wurden Kalium-oxo-phenylacetat (225,9 mg, 1.2 mmol), Bis(1,1,1,5,5,5-hexafluoracetylacetonato)palladium (5,2 mg, 0.01 mmol) und Kupfer(I)bromid (21,5 mg, 0,15 mmol) unter Stickstoff vorgelegt und mit einer Lösung von 4-Bromtoluol (171,0 mg, 1,0 mmol), Tris(o-tolyl)phosphin (6,1 mg, 0.02 mmol) und 1,10-Phenanthrolin (27 mg, 0,15 mmol) in 1,5 mL NMP und 0,5 mL Chinolin versetzt. Die Reaktionsmischung wurde für 16 Stunden unter Rückfluss erhitzt (170 °C), nach dem Abkühlen wurden 20 mL 1 M Salzsäure zugegeben und die wässrigen Phasen dreimal mit Ethylacetat extrahiert. Die vereinigten organischen Phasen wurden eingeengt und der Rückstand chromatographisch gereinigt. Das Produkt wurde als gelbes Öl erhalten (163 mg, 83% Ausb.). Die spektroskopischen Daten entsprechen denen von 4-Methylbenzophenon.

### Beispiele 3 bis 28

Es wurden zu den Beispielen 3 bis 28 nachstehende Katalysatorsysteme gefunden: 15 Mol-% Kupfer(I)bromid, 15 mol% 1,10-Phenanthrolin, 2 mol% (*o*-Tol)₃P und 1 mol% Pd(F₆-acac)₂, bei 170°C in einem NMP/Chinolingemisch, das eine Vielzahl von Arylbromiden mit unterschiedlichen alpha-Oxocarbonsäuren zu Ketonen erfolgreich umsetzt (Tabelle 1).

### Ablauf der Reaktion:

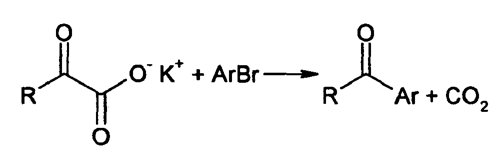

| **Bsp.** | **Produkt** | **Ausb. /%** | **Bsp.** | **Produkt** | **Ausb. /%** |
|---|---|---|---|---|---|
| 3 | 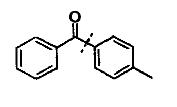 | 83 | 16 | 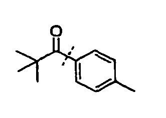 | 90 |
| 4 | 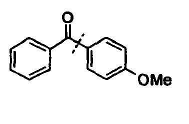 | 82 | 17 | 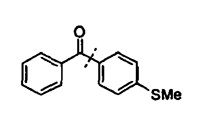 | 67 |
| 5 | 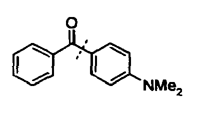 | 59 | 18 | 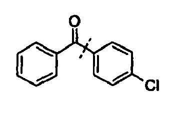 | 56 |
| 6 | 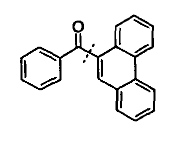 | 83 | 19 | 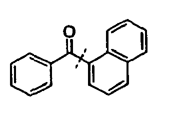 | 82 |
| 7 | 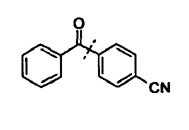 | 99 | 20 | 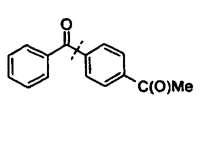 | 78 |
| 8 | 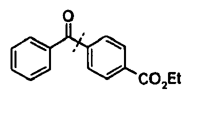 | 70 | 21 | 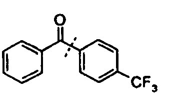 | 72 |
| 9 | 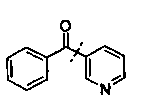 | 57 | 22 | 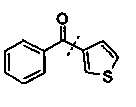 | 50 |
| 10 | 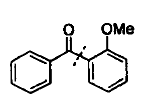 | 73 | 23 | 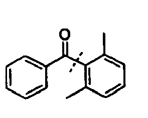 | 26 |
| 11 | 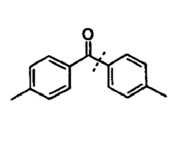 | 72 | 24 | 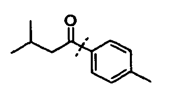 | 69 |
| 12 | 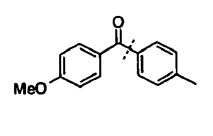 | 64 | 25 | 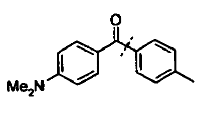 | 59 |
| 13 | 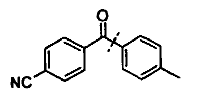 | 96 | 26 | 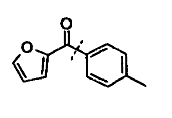 | 51 |
| 14 | 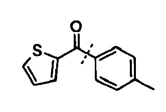 | 45 | 27 | 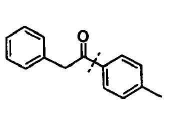 | 34 |
| 15 | 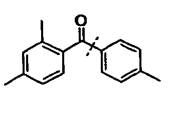 | 52 | 28 | 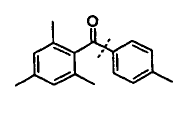 | 5 |

Der Substituent aus der alpha-Oxocarbonsäure ist in Tabelle 1 auf der linken Seite der Carbonylgruppe gezeichnet.

Aus der Tabelle 1 kann man erkennen, dass die alpha-Oxocarbonsäuren sowohl mit elektronenreichen wie -armen Aryl- und Heteroarylbromiden in guten Ausbeuten zu den Phenylketonen reagieren, wobei viele funktionelle Gruppen toleriert werden, u.a. Ester, Ketone und Nitrile. Andererseits ließ sich 4-Bromtoluol mit diversen alkyl-, aryl- und heteroarylsubstituierten alpha-Oxocarbonsäuren in guten Ausbeuten kuppeln und daher die breit anwendbare neue Synthesemethode zu Herstellung von Arylketonen belegt.

## Patentansprüche

1. Verfahren zur Herstellung von Ketonen der allgemeinen Formel (III) 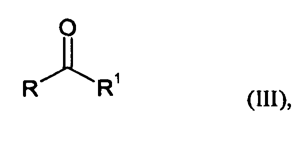 wobei
R ein gegebenenfalls substituierter carbocyclischer aromatischer Rest mit 6 bis 24 Kohlenstoffatomen oder ein gegebenenfalls substituierter Alkylrest oder ein gegebenenfalls substituierter heteroaromatischer Rest mit 5 bis 24 Kohlenstoffatomen ist und
R¹ ein gegebenenfalls substituierter carbocyclischer aromatischer Rest mit 6 bis 24 Kohlenstoffatomen oder ein gegebenenfalls substituierter heteroaromatischer Rest mit 5 bis 24 Kohlenstoffatomen ist,
durch Umsetzung von alpha-Oxocarboxylaten der allgemeinen Formel (I) 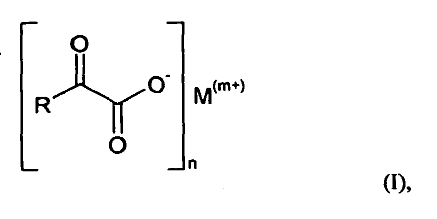 wobei
n und m eine Zahl im Bereich von 1 bis 6 ist,
M^{(m+)} ein Kation ist und
R die für Formel (III) angegebene Bedeutung hat,
mit Arylbromiden der allgemeinen Formel (II)
R¹-Br (II)
wobei
R¹ die für Formel (III) angegebene Bedeutung hat,
**dadurch gekennzeichnet, dass** als Katalysator eine Kombination aus Kupfer(I)bromid mit 1,10-Phenanthrolin als Ligand und Bis(1,1,1,5,5,5-hexafluoroacetylacetonato)palladium mit Tris(o-tolyl)phosphin als Ligand eingesetzt wird.

2. Verfahren nach Anspruch 1, wobei von beiden Katalysatoren unabhängig voneinander Mengen von 0,001 mol% bis 100 mol% bezogen auf die Verbindung der Formel (II) eingesetzt werden.

3. Verfahren nach einem der Ansprüche 1 bis 2, **dadurch gekennzeichnet, dass** der Rest R in Verbindung der Formel (III) ein Phenyl-, Cyanophenyl- oder tert.-Butylrest ist.

4. Verfahren nach einem der Ansprüche 1 bis 3, **dadurch gekennzeichnet, dass** der Rest R¹ in der Verbindung der Formel (III) ein Tolyl-, Cyanophenyl- oder Methoxyphenylrest ist.

## Claims

1. Process for preparing ketones of the general formula (III) 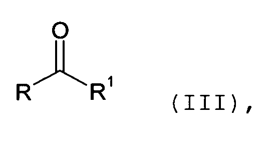 where
R is an optionally substituted carbocyclic aromatic radical having 6 to 24 carbon atoms or an optionally substituted alkyl radical or an optionally substituted heteroaromatic radical having 5 to 24 carbon atoms, and
R¹ is an optionally substituted carbocyclic aromatic radical having 6 to 24 carbon atoms or an optionally substituted heteroaromatic radical having 5 to 24 carbon atoms,
by reacting alpha-oxo carboxylates of the general formula (I) 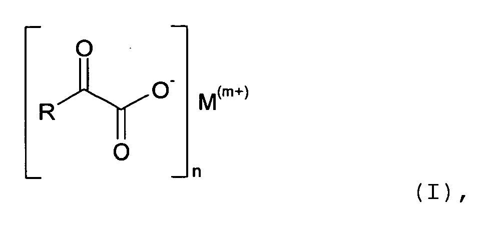 where
n and m is a number in the range from 1 to 6,
M^{(m+)} is a cation, and
R has the meaning indicated for formula (III),
with aryl bromides of the general formula (II)
R¹-Br (II)
where
R¹ has the meaning indicated for formula (III), **characterized in that** a combination of copper(I) bromide with 1,10-phenanthroline as ligand and bis(1,1,1,5,5,5-hexafluoroacetylacetonato)-palladium with tris(o-tolyl)phosphine as ligand is employed as catalyst.

2. Process according to Claim 1, where the amounts of the two catalysts employed independently of one another are from 0.001 mol% to 100 mol% based on the compound of the formula (II).

3. Process according to either of Claims 1 and 2, **characterized in that** the radical R in compound of the formula (III) is a phenyl, cyanophenyl or tert-butyl radical.

4. Process according to any of Claims 1 to 3, **characterized in that** the radical R¹ in the compound of the formula (III) is a tolyl, cyanophenyl or methoxyphenyl radical.

## Revendications

1. Procédé pour la préparation de cétones de formule générale (III) 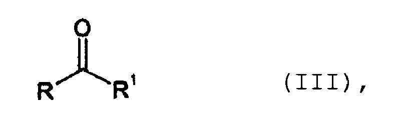 dans laquelle
R est un radical aromatique carbocyclique éventuellement substitué ayant de 6 à 24 atomes de carbone ou un radical alkyle éventuellement substitué ou un radical hétéroaromatique éventuellement substitué ayant de 5 à 24 atomes de carbone et
R¹ est un radical aromatique carbocyclique éventuellement substitué ayant de 6 à 24 atomes de carbone ou un radical hétéroaromatique éventuellement substitué ayant de 5 à 24 atomes de carbone,
par mise en réaction d'alpha-oxocarboxylates de formule générale (I) 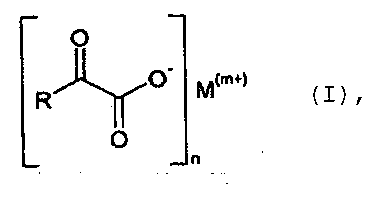 dans laquelle
n et m représentent un nombre dans la plage allant de 1 à 6,
M^{(m+)} est un cation et
R a la signification indiquée pour la formule (III),
avec des bromures d'aryle de formule générale (II)
R¹-Br (II)
R¹ ayant la signification indiquée pour la formule (III)
**caractérisé en ce qu'**on utilise comme catalyseur une association de bromure de cuivre(I) avec de la 1,10-phénanthroline en tant que ligand et de bis(1,1,1,5,5,5-hexafluoroacétylacétonato)palladium avec de la tris(o-tolyl)phosphine en tant que ligand.

2. Procédé selon la revendication 1, dans lequel on utilise des quantités des deux catalyseurs, indépendamment l'un de l'autre, de 0,001 % en moles à 100 % en moles, par rapport au composé de formule (II).

3. Procédé selon l'une quelconque des revendications 1 et 2, **caractérisé en ce que** le radical R dans le composé de formule (III) est un radical phényle, cyanophényle ou tert-butyle.

4. Procédé selon l'une quelconque des revendications 1 à 3, **caractérisé en ce que** le radical R¹ dans le composé de formule (III) est un radical tolyle, cyanophényle ou méthoxyphényle.
